# FASCICULE DE BREVET EUROPEEN

(11) **EP 0 652 742 B1**
(45) Date de publication et mention de la délivrance du brevet: **27.08.1997**
(21) Numéro de dépôt: 94917046.8
(22) Date de dépôt: 24.05.1994
(51) Int. Cl.: A61K 7/13, C07C 215/76

(54) **UTILISATION POUR LA TEINTURE DES FIBRES KERATINIQUES DE PARA-AMINOPHENOLS 3-SUBSTITUES ET NOUVEAUX PARA-AMINOPHENOLS 3-SUBSTITUES**
VERWENDUNG VON 3-SUBSTITUIERTEN PARA-AMINOPHENOLEN ZUM FÄRBEN VON KERATINFASERN UND NEUE SUBSTITUIERTE 3-PARA-AMINOPHENOLE
USE OF 3-SUBSTITUTED PARA-AMINOPHENOLS FOR DYEING KERATINOUS FIBRES AND NOVEL SUBSTITUTED 3-PARA-AMINOPHENOLS

(30) Priorité: 25.05.1993 FR 9306231
(43) Date de publication de la demande: 17.05.1995
(73) Titulaire: L'OREAL, 75008 Paris (FR)
(72) Inventeur: LAGRANGE, Alain, F-77700 Coupvray (FR); VANDENBOSCHE, Jean-Jacques, F-93270 Sevran (FR); COTTERET, Jean, F-78480 Verneuil-sur-Seine (FR); AUDOUSSET, Marie-Pascale, F-92300 Levallois-Perret (FR)
(74) Mandataire: Casalonga, Axel
(86) Numéro de dépôt international: FR9400606
(87) Numéro de publication internationale: WO9427564

(56) Documents cités:
- EP-A- 0 241 716
- EP-A- 0 331 144
- FR-A- 2 421 870
- US-A- 5 034 014
- PATENT ABSTRACTS OF JAPAN vol. 13, no. 205 (C-595) 15 Mai 1989 & JP,A,01 022 972 (KAO CORP) 25 Janvier 1989

## Description

L'invention est relative à l'utilisation pour la teinture des fibres kératiniques, et en particulier des cheveux humains, de para-aminophénols 3-substitués dans des compositions tinctoriales, ces compositions étant utilisées pour la coloration dite coloration d'oxydation ou teinture permanente et à des para-aminophénols 3-substitués.

La teinture des fibres kératiniques par la coloration dite d'oxydation utilise des précurseurs de colorants d'oxydation appelés également bases d'oxydation qui sont incolores, mais au contact d'un oxydant, développent dans les fibres kératiniques une coloration durable.

On connaît comme précurseurs de colorants d'oxydation, les para-phénylènediamines, les ortho-phénylènediamines, les para-aminophénols, les ortho-aminophénols, substitués ou non substitués. Ces précurseurs de colorants peuvent être mélangés avec un ou plusieurs composés appelés "coupleurs", qui permettent de faire varier les nuances obtenues avec les précurseurs de colorants. Ces coupleurs sont généralement choisis parmi les métaphénylènediamines, les métaaminophénols, les métadiphénols et les phénols.

Dans le domaine de la teinture des fibres kératiniques et en particulier des cheveux humains, on est à la recherche de précurseurs de colorants d'oxydation qui, associés à des coupleurs, permettent de conférer aux cheveux une coloration ayant une résistance satisfaisante à la lumière, au lavage, aux intempéries, à la transpiration et aux différents traitements que peuvent subir les cheveux. De plus, ces précurseurs de colorants d'oxydation doivent bénéficier d'une bonne innocuité.

Le document FR-A-2 421 870 décrit des compositions tinctoriales contenant des métaphénylènediamines.

La demanderesse vient de découvrir, ce qui fait l'objet de l'invention, que l'utilisation de certains para-aminophénols 3-substitués en tant que précurseurs de colorants d'oxydation dans des compositions tinctoriales pour fibres kératiniques, permettaient d'obtenir sur ces dernières et en particulier sur les cheveux humains, des colorations présentant une bonne résistance à la lumière, au lavage, aux intempéries, à la transpiration et aux différents traitements que peuvent subir les cheveux, et en particulier à la lumière et aux intempéries. Les couleurs obtenues sont peu sélectives, c'est-à-dire sensiblement identiques sur cheveux naturels et sur cheveux sensibilisés par un traitement tel qu'une décoloration ou une permanente, ce qui fait qu'on obtient un bon unisson de coloration de la racine, naturelle, à la pointe, sensibilisée, du cheveu. Ces para-amino-phénols 3-substitués présentent en outre une bonne innocuité.

L'invention a pour objet l'utilisation des para-aminophénols 3-substitués de formule (I) définie ci-après pour la teinture des fibres kératiniques et en particulier des cheveux humains.

L'invention a également pour objet des compositions tinctoriales pour fibres kératiniques et en particulier pour cheveux humains contenant, dans un milieu aqueux approprié pour la teinture, au moins un para-aminophénol 3-substitué de formule (I) : 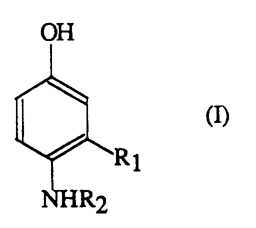 dans laquelle R₁ représente un radical alkyle en C₁-C₄, un radical alcényle en C₂-C₄, un radical monohydroxyalkyle en C₁-C₆, polyhydroxyalkyle en C₂-C₆, alcoxyalkyle en C₂-C₆, nitrile, cyanoalkyle en C₁-C₄, halogénoalkyle en C₁-C₄ et de préférence fluoroalkyle en C₁-C₄, aminoalkyle de formule : 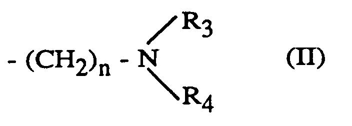 dans laquelle :
n est un nombre entier compris entre 1 et 6 inclus;
R₃ et R₄ identiques ou différents, représentent un atome d'hydrogène ou un radical alkyle en C₁-C₄, hydroxyalkyle en C₁-C₄, acyle en C₁-C₆;
R₂ représente un atome d'hydrogène, un radical alkyle en C₁-C₆, un radical monohydroxyalkyle en C₁-C₆, polyhydroxyalkyle en C₂-C₆, sous réserve que :
   lorsque R₂ est hydrogène, R₁ ne représente pas méthyle ou trifluorométhyle,
   ou l'un de ses sels d'addition avec un acide.

Parmi les composés de formule (I) ci-dessus, on peut citer plus particulièrement :
- le 3-éthyl p-aminophénol
- le 3-hydroxyméthyl p-aminophénol
- le 3-cyanométhyl p-aminophénol
- le 3-tert.-butyl p-aminophénol
- le 3-(β-méthoxyéthyl)p-aminophénol
- le 3-(β-éthoxyéthyl)p-aminophénol.

Un autre objet de l'invention porte sur le procédé de coloration des fibres kératiniques et en particulier des cheveux humains, mettant en oeuvre une composition tinctoriale telle que définie ci-dessus, mélangée à un agent oxydant.

L'invention a également pour objet de nouveaux para-aminophénols 3-substitués.

D'autres objets de l'invention apparaîtront à la lecture de la description et des exemples qui suivent.

Les nouveaux para-aminophénols 3-substitués selon l'invention ont pour formule : 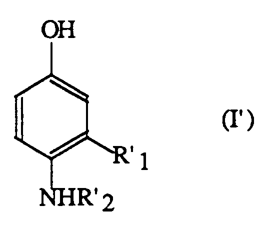 dans laquelle R'₁ et R'₂ ont les significations indiquées pour R₁ et R₂, sous réserve que :
- lorsque R'₂ désigne un atome d'hydrogène, R'₁ ne représente pas un radical alkyle, vinyle, dichlorométhyle ou trifluorométhyle;
- lorsque R'₁ désigne le radical méthyle, R'₂ ne peut désigner le radical éthyle.

Les composés, selon l'invention, peuvent aussi être constitués par les sels d'addition avec un acide des composés (I') ci-dessus.

Parmi les para-aminophénols 3-substitués de formule (I'), on peut citer :
- le 3-hydroxyméthyl p-aminophénol
- le 3-cyanométhyl p-aminophénol
- le 3-(β-méthoxyéthyl)p-aminophénol
- le 3-(β-éthoxyéthyl)p-aminophénol,
et plus particulièrement le 3-hydroxyméthyl p-aminophénol.

Dans les formules (I) ou (I') ci-dessus, le radical alkyle désigne de préférence les radicaux méthyle, éthyle, propyle, isopropyle, butyle, sec-butyle, tert-butyle, le radical mono- ou polyhydroxyalkyle désigne de préférence :
- CH₂OH , - CH₂-CH₂OH , - CH₂CHOH-CH₂-OH , - CH₂-CHOH-CH₃ ;
le radical alcoxyalkyle désigne de préférence :
- CH₂CH₂OCH₃ , - CH₂CH₂OC₂H₅ ;
le radical aminoalkyle désigne de préférence :
- CH₂-CH₂-NH₂ , - CH₂-CH₂-NHCH₃ , 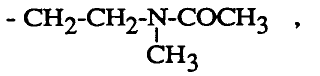 - CH₂-CH₂-NHCOCH₃ ;
lorsque les radicaux R₃ et R₄ représentent un radical acyle, celui-ci désigne de préférence les radicaux formyle, acétyle et propionyle.

Parmi les radicaux fluoroalkyle, on peut citer le radical trifluorométhyle et le radical trifluoroéthyle,

Les sels d'acides correspondant aux composés de formules (I) ou (I') sont choisis de préférence parmi les chlorhydrates, les sulfates et les bromhydrates.

Les composés nouveaux de formule (I') dans lesquels R'₂=H, sont préparés par l'un des procédés suivants :
1) Nitrosation du phénol 3-substitué correspondant au p-aminophénol que l'on souhaite obtenir, à l'aide de nitrite de sodium ou de potassium ou de métal alcalino-terreux en milieu alcalin, puis hydrogénation du groupement nitroso par l'hydrosulfite de sodium en milieu alcalin en présence d'hydrogène et de métaux de transition tels que le platine ou le palladium.
   Ce procédé de préparation correspond au schéma réactionnel ci-après : 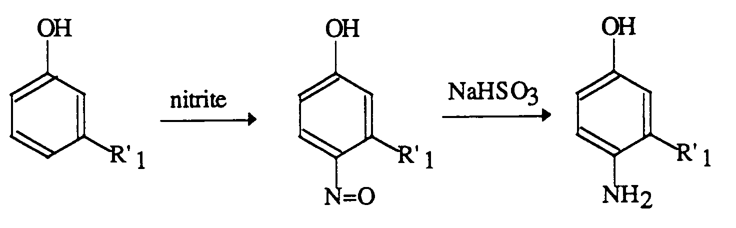
2) Réduction du groupe nitro d'un nitrobenzène 2-substitué pour obtenir une arylhydroxylamine, soit par de l'aluminium en milieu aqueux acidifié, soit en présence d'hydrogène en milieu aqueux acidifié et d'un catalyseur de type platine, puis réarrangement de BAMBERGER en présence d'un acide selon le schéma réactionnel décrit dans "Advanced Organic Chemistry", p. 606. 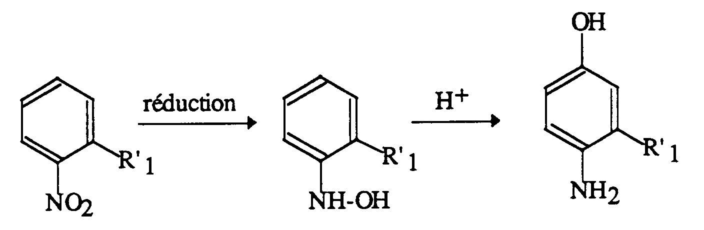
3) Condensation d'un sel de diazonium de l'acide p-sulfanilique sur le phénol 3-substitué correspondant au p-aminophénol que l'on souhaite obtenir, puis réduction du composé azoïque obtenu par l'hydrosulfite de sodium en milieu alcalin en présence d'hydrogène et de métaux de transition tels que le platine ou le palladium, selon le schéma réactionnel suivant : 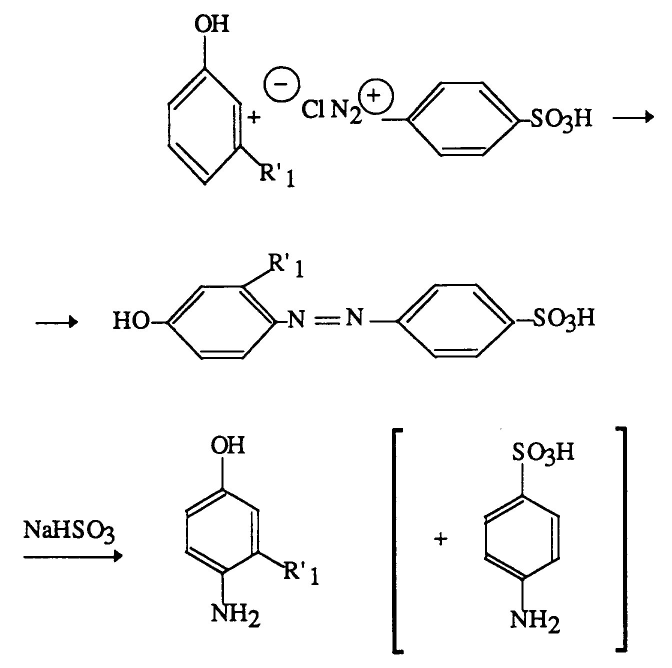
4) Réduction du groupe nitro et clivage du groupe benzyloxy (BzO) d'un composé de formule : 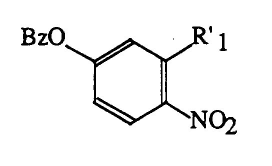 sous pression d'hydrogène en présence de palladium sur charbon dans du cyclohexène.

Les composés de formule (I') dans lesquels R'₂ n'est pas, un atome d'hydrogène, sont obtenus par les méthodes classiques d'alkylation et d'hydroxyalkylation des amines aromatiques.

Dans les compositions tinctoriales selon l'invention, les composés de formule (I) sont généralement employés en présence d'un coupleur choisi parmi les métadiphénols, les métaaminophénols, les métaphénylènediamines de formule (III) : 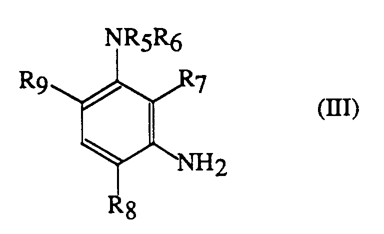 dans laquelle :
R₅ et R₆ désignent, indépendamment l'un de l'autre, un atome d'hydrogène, un groupement alkyle en C₁-C₄ ou hydroxyalkyle en C₁-C₄ ;
R₇ désigne un atome d'hydrogène ou un groupement alkyle ou alcoxy en C₁-C₄ ;
R₈ désigne un atome d'hydrogène ou un groupement alkyle en C₁-C₄, hydroxyalcoxy en C₁-C₄ ou alcoxy en C₁-C₄ ;
R₉ désigne un atome d'hydrogène ou d'halogène ou un groupement alkyle en C₁-C₄, alcoxy en C₁-C₄, hydroxyalkyle en C₁-C₄, hydroxyalcoxy en C₁-C₄, polyhydroxyalcoxy en C₂-C₄, carboxyalcoxy en C₁-C₄, 2',4'-diaminophénoxyalcoxy en C₁-C₄ ou aminoalcoxy en C₁-C₄ ;
   sous réserve que si R₉ désigne carboxyalcoxy ou 2',4'-diaminophénoxyalcoxy, alors R₅, R₆, R₇ et R₈ désignent de l'hydrogène,
   ainsi que leurs sels,
   les métaacylaminophénols, les métauréidophénols, les métacarbalcoxyaminophénols, l'α-naphtol, les dérivés indoliques, les coupleurs possédant un groupement méthylène actif, tels que les composés β-cétoniques et les pyrazolones.

Parmi ces coupleurs on peut plus particulièrement citer le 2, 4-dihydroxyphénoxyéthanol, le 2,4-dihydroxyanisole, le métaaminophénol, le monométhyléther de résorcine, la résorcine, la 2-méthyl-résorcine, le 2-méthyl 5-aminophénol, le 2-méthyl 5-N-(β-hydroxyéthyl) aminophénol, le 2-méthyl 5-N-(β-mésylaminoéthyl)aminophénol, le 2,6-diméthyl 3-aminophénol, la 6-hydroxybenzomorpholine, le 2,4-diaminoanisole, le 2,4-diaminophénoxyéthanol, la 6-aminobenzomorpholine, le [2-N-(β-hydroxyéthyl) amino 4-amino]-phénoxyéthanol, le 2-hydroxy 4-N-(β-hydroxyéthyl)aminoanisole, le 2-amino 4-N-(β-hydroxyéthyl) amino anisole, le (2,4-diamino)phényl-β,γ-dihydroxypropyléther, le (2,4-diamino)phényl-α,β-dihydroxypropyléther, le 1-[2',4'-diaminophénoxypropyloxy]2,4-diaminobenzène, l'acide 2,4-diaminophénoxyacétique, la 2,4-diaminophénoxyéthylamine, le 1,3-diméthoxy 2,4-diaminobenzène, le 1,3,5-triméthoxy 2,4-diaminobenzène, le 1-amino 3,4-méthylènedioxybenzène, le 1-hydroxy 3,4-méthylènedioxybenzène, le 2-chloro 6-méthyl 3-aminophénol, le 2-méthyl 3-aminophénol, le 2-chlororésorcinol, la 6-méthoxy 3-hydroxyéthylaminoaniline, le 1-éthoxy 2-bis(β-hydroxyéthyl)amino 4-aminobenzène, le 3-diéthylaminophénol, le 1,3-dihydroxy 2-méthylbenzène, le 1-hydroxy 2,4-dichloro 3-aminobenzène, le 4,6-bis-(β-hydroxyéthoxy)1,3-diaminobenzène, le 4-méthyl 6-éthoxy 1,3-diaminobenzène, le 4-chloro 6-méthyl 3-aminophénol, le 6-chloro 3-trifluoroéthylaminophénol, le 6-hydroxyindole, le 7-hydroxyindole, le 4-hydroxyindole, le 7-aminoindole, le 5,6-dihydroxyindole, ainsi que leurs dérivés tels que décrits dans les brevets FR-2.636.236, FR-2.654.335, FR-2.654.336, FR-2.659.228, FR-2.664.304, FR-2.664.305 et FR-2.671.722.

En plus des para-aminophénols 3-substitués de formule (I), les compositions tinctoriales selon l'invention peuvent contenir d'autres précurseurs de colorants d'oxydation para et/ou ortho connus en eux-mêmes.

Ces précurseurs de colorants d'oxydation de type ortho ou para sont des composés benzéniques ou hétérocycliques qui comportent deux groupements fonctionnels amino ou hydroxy et amino, en position ortho ou para l'un par rapport à l'autre.

Les précurseurs de colorants d'oxydation de type ortho ou para peuvent être choisis parmi les paraphénylènediamines, les paraaminophénols différents de ceux de formule (I), les précurseurs hétérocycliques para dérivés de la pyridine ou de la pyrimidine, tels que la 2,5-diaminopyridine, la 2-hydroxy 5-aminopyridine, la 2,4,5,6-tétraminonopyrimidine, le 4,5-diamino 1-méthylpyrazole, la 2-diméthylamino 4,5,6-triaminopyrimidine, les orthoaminophénols et les bases dites "doubles".

A titre de paraphénylènediamines, on peut plus particulièrement citer les composés répondant à la formule (IV) : 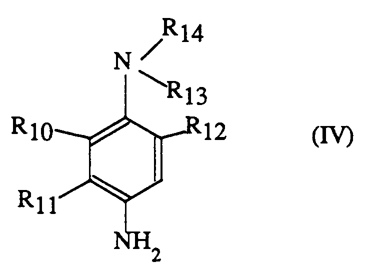 dans laquelle :
R₁₀, R₁₁, R₁₂, identiques ou différents, représentent un atome d'hydrogène ou d'halogène, un radical alkyle, un radical alcoxy, un radical carboxy, sulfo ou hydroxyalkyle en C₁-C₄ ;
R₁₃ et R₁₄, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, sulfoalkyle, pipéridinoalkyle, morpholinoalkyle, ou phényle éventuellement substitué en para par un groupement amino; ou bien R₁₃ et R₁₄ forment conjointement avec l'atome d'azote auquel ils sont liés, un hétérocycle pipéridino ou morpholino, sous réserve que R₁₀ ou R₁₂ représente un atome d'hydrogène lorsque R₁₃ et R₁₄ ne représentent pas un atome d'hydrogène, ainsi que les sels de ces composés. Ces radicaux alkyle ou alcoxy ont de préférence 1 à 4 atomes de carbone et désignent notamment les radicaux méthyle, éthyle, propyle, méthoxy et éthoxy.

Parmi les composés de formule (IV), on peut plus particulièrement citer la paraphénylènediamine, la paratoluylènediamine, la méthoxyparaphénylènediamine, la chloroparaphénylènediamine, la 2,3-diméthylparaphénylènediamine, la 2,6-diméthylparaphénylènediamine, la 2,6-diéthylparaphénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthylparaphénylènediamine, la N,N-diéthylparaphénylènediamine, la N,N-dipropylparaphénylènediamine, la 3-méthyl 4-amino N,N-diéthylaniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl, carbamylméthyl)-aniline, la 3-méthyl 4-amino N,N-(éthyl, carbamylméthyl)aniline, la 4-amino N,N-(éthyl, β-pipéridinoéthyl) aniline, la 3-méthyl 4-amino N,N-(éthyl, β-pipéridinoéthyl)aniline, la 4-amino N,N-(éthyl, β-morpholinoéthyl)aniline, la 3-méthyl 4-amino N,N-(éthyl,β-morpholinoéthyl)aniline, la 4-amino N,N-(éthyl,β-acétylaminoéthyl) aniline, la 4-amino N-(β-méthoxyéthyl) aniline, la 3-méthyl 4-amino N,N-(éthyl, β-acétylaminoéthyl) aniline, la 4-amino N,N-(éthyl, β-mésylaminoéthyl) aniline, la 3-méthyl 4-amino N,N-(éthyl, β-mésylaminoéthyl) aniline, la 4-amino N,N-(éthyl, β- sulfoéthyl)aniline, la 3-méthyl 4-amino N,N-(éthyl, β-sulfoéthyl) aniline, la N-[(4'-amino)phényl]-morpholine, la N-[(4'-amino)phényl]pipéridine, la 2-β-hydroxyéthylparaphéylènediamine, la fluoroparaphénylènediamine, la carboxyparaphénylènediamine, la sulfoparaphénylènediamine, la 2-isopropylparaphénylènediamine, la 2-n-propylparaphénylènediamine, l'hydroxy-2-n-propylparaphénylènediamine, la 2-hydroxyméthylparaphénylènediamine, la N,N-diméthyl 3-méthylparaphéylènediamine, la N,N-(éthyl,β-hydroxyéthyl)paraphénylenèdiamine, la N-(dihydroxypropyl)paraphénylènediamine, la N-4'-aminophénylparaphénylènediamine, la N-phénylparaphénylènediamine.

Ces paraphénylènediamines peuvent être utilisées soit sous forme de base libre, soit sous forme de sels, tels que chlorhydrate, bromohydrate ou sulfate.

Parmi les p-aminophénols différents de ceux de formule (I), on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2,5-diméthyl 4-aminophénol, le 2-hydroxyméthyl 4-aminophénol, le 2-(β-hydroxyéthyl)4-aminophénol, le 2-méthoxy 4-aminophénol, le 3-méthoxy 4-aminophénol, le 3-(β-hydroxyéthoxy)4-aminophénol, le 2-aminométhyl 4-aminophénol, le 2-β-hydroxyéthylaminométhyl 4-aminophénol, et ceux de formule (V) ci-après : 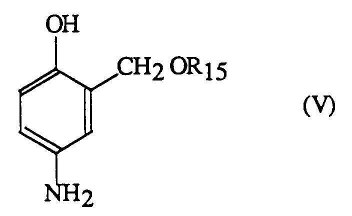 dans laquelle R₁₅ représente un radical alkyle en C₁-C₆ hydroxyalkyle en C₁-C₆, polyhydroxyalkyle en C₂-C₆ ou halogénoalkyle en C₁-C₆, aminoalkyle en C₂-C₄, aminoalkyle en C₂-C₄ dont l'amine peut être monosubstituée ou disubstituée par un groupement alkyle en C₁-C₄ ou substituée par un groupement dihydroxyalkyle en C₃-C₄, ainsi que leurs sels.

Parmi Ces composés de formule (V), on peut en particulier citer :
- le 2-méthoxyméthyl 4-aminophénol,
- le 2-éthoxyméthyl 4-aminophénol,
- le 2-n-propyloxyméthyl 4-aminophénol,
- le 2-isopropyloxyméthyl 4-aminophénol,
- le 2-(β-hydroxyéthoxy)méthyl 4-aminophénol,
- le 2-[(2',2',2'-trifluoroéthoxy)méthyl]4-aminophénol,
ainsi que leurs sels.

Les bases dites "doubles" sont des bis-phénylalkylènediamines, répondant à la formule : 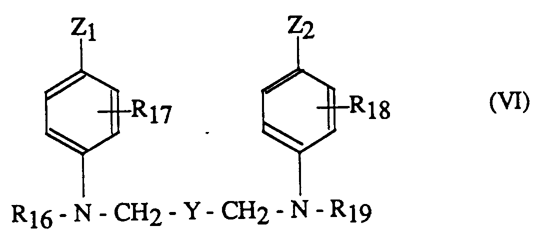 dans laquelle :
Z₁ et Z₂, identiques ou différents, représentent des groupements hydroxyle ou NHR₂₀, où R₂₀ désigne un atome d'hydrogène ou un radical alkyle inférieur ;
R₁₇ et R₁₈, identiques ou différents, représentent soit des atomes d'hydrogène, soit des atomes d'halogène, soit encore des radicaux alkyle ;
R₁₆ et R₁₉, identiques ou différents, représentent un atome d'hydrogène, un radical alkyle, hydroxyalkyle ou aminoalkyle, dont le reste amino peut être substitué; Y représente un radical pris dans le groupe constitué par les radicaux suivants :
   -(CH₂)ₙ-, -(CH₂)ₘ-O-(CH₂)ₘ-,
   -(CH₂)_{q}-CHOH-(CH₂)_{q}-, 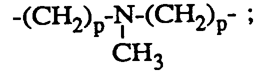 dans lesquels n est un nombre entier compris entre 0 et 8 et m, q et p sont des nombres entiers compris entre 0 et 4, cette base pouvant se présenter également sous forme de ses sels d'addition avec des acides.

Les radicaux alkyle ou alcoxy ci-dessus indiqués désignent de préférence un groupement ayant 1 à 4 atomes de carbone et notamment méthyle, éthyle, propyle, méthoxy et éthoxy.

Parmi les composés de formule (VI), on peut citer le N,N'-bis-(β-hydroxyéthyl)N,N'-bis-(4'-aminophényl) 1,3-diamino 2-propanol, la N,N'-bis-(β-hydroxyéthyl)N,N'-bis-(4'-aminophényl)éthylènediamine, la N,N'-bis-(4-aminophényl)tétraméthylènediamine, la N,N'-bis-(β-hydroxyéthyl) N,N'-bis-(4-aminophényl)tétraméthylènediamine, la N,N'-bis-(4-méthylaminophényl)tétraméthylènediamine, la N,N'-bis-(éthyl) N,N'-bis-(4'-amino 3'-méthylphényl)éthylènediamine.

Parmi les orthoaminophénols, on peut citer plus particulièrement l'ortho-aminophénol, le 6-méthyl 1-hydroxy 2-aminobenzène, le 4-méthyl 1-amino 2-hydroxybenzène, le 4-acétylamino 1-amino 2-hydroxybenzène.

Selon une première forme de réalisation particulière de l'invention, la composition contient l'association suivante :
- au moins le 3-éthyl p-aminophénol ou l'un de ses sels, à titre de précurseur de colorant d'oxydation;
- au moins le 2-méthyl 5-aminophénol et/ou le 2-méthyl 5-N-(β-hydroxyéthyl)aminophénol ou l'un de leurs sels, à titre de coupleurs;
- au moins une métaphénylènediamine de formule (III) ci-dessus définie, à titre de coupleur additionnel.

Selon cette première forme de réalisation de l'invention, on préfère parmi les métaphénylènediamines de formule (III) ci-dessus, celles pour lesquelles les groupements R₅ à R₈ désignent hydrogène et R₉ désigne le groupement hydroxyalcoxy en C₁-C₄ ou polyhydroxyalcoxy en C₂-C₄.

Parmi les radicaux hydroxyalcoxy en C₁-C₄, on préfère plus particulièrement le radical β-hydroxyéthyloxy.

Parmi les radicaux polyhydroxyalcoxy en C₂-C₄, on préfère plus particulièrement le radical 1,2-dihydroxypropyloxy.

On peut tout particulièrement citer :
- le 2,4-diaminophénoxyéthanol,
- le (2,4-diamino)phényl-α,β-dihydroxypropyléther,
- le 1-[2',4'-diaminophénoxypropyloxy]2,4-diaminobenzène,
- le 2-amino 4-N-(β-hydroxyéthyl)aminoanisole,
- l'acide 2,4-diaminophénoxyacétique,
- le 4,6-bis-(β-hydroxyéthoxy)1,3-diaminobenzène,
ainsi que leurs sels.

On obtient des colorations aux nuances cuivrées et particulièrement résistantes aux lavages.

Selon une seconde forme de réalisation particulière de l'invention, la composition contient l'association suivante :
- au moins le 3-éthyl p-aminophénol ou l'un de ses sels, à titre de précurseur de colorant d'oxydation;
- au moins le 2-méthyl 5-aminophénol et/ou le 2-méthyl 5-N-(β-hydroxyéthyl)aminophénol ou l'un de leurs sels, à titre de coupleurs;
- au moins un précurseur de colorant d'oxydation additionnel, choisi parmi :
   . les orthoaminophénols décrits précédemment,
   . les paraphénylènediamines de formule (IV),
   . les bis-phénylalkylènediamines de formule (VI),
   . les paraaminophénols de formule (V).

Selon cette seconde forme de réalisation de l'invention et lorsque l'on utilise un orthoaminophénol, on emploie de préférence l'orthoaminophénol ou le 4-acétylamino 1-amino 2-hydroxybenzène ou leurs sels. On obtient des colorations aux nuances cuivrées et particulièrement résistantes aux lavages.

Selon cette seconde forme de réalisation de l'invention et lorsque l'on utilise une paraphénylènediamine de formule (IV), on emploie de préférence :
- la paraphénylènediamine,
- la paratoluylènediamine,
- la 2,6-diméthyl p-phénylènediamine,
- la 2-β-hydroxyéthyl p-phénylènediamine,
- la 2-isopropyl p-phénylènediamine,
- la N,N-di(β-hydroxyéthyl)p-phénylènediamine,
- la 4-amino N-(β-méthoxyéthyl)aniline,
ou leurs sels.

On obtient des colorations aux nuances rouges ou cuivrées et particulièrement résistantes à la transpiration.

Selon cette seconde forme de réalisation de l'invention et lorsque l'on utilise une bis-phénylalkylènediamine, on emploie de préférence le N,N'-bis-(β-hydroxyéthyl)N,N'-bis-(4'-aminophényl)1,3-diamino 2-propanol. On obtient également des colorations rouges ou cuivrées et particulièrement résistantes à la transpiration.

Selon cette seconde forme de réalisation de l'invention et lorsque l'on utilise un paraaminophénol de formule (V), on utilise de préférence le 2-méthoxyméthyl 4-aminophénol ou son sel d'addition avec un acide. On obtient ainsi des colorations aux nuances rouges ou cuivrées, présentant une bonne résistance aux différents traitements que peuvent subir les cheveux.

On peut rajouter à ces compositions, comme cela est bien connu dans l'état de la technique, notamment en vue de nuancer ou d'enrichir en reflet les colorations apportées par les précurseurs de colorants d'oxydation, des colorants directs tels que des colorants azoïques, anthraquinoniques ou les dérivés nitrés de la série benzénique.

L'ensemble des précurseurs de colorants par oxydation de type para et/ou ortho, ainsi que les coupleurs utilisés dans les compositions tinctoriales conformes à l'invention, représente de préférence de 0,3 à 7 % en poids par rapport au poids total de la dite composition. La concentration en composé de formule (I) peut varier entre 0,05 et 3,5 % en poids du poids total de la composition.

Les compositions tinctoriales conformes à l'invention contiennent également dans leur forme de réalisation préférée, des agents tensioactifs anioniques, cationiques, non-ioniques, amphotères ou leurs mélanges. Parmi Ces agents tensio-actifs, on peut citer les alkylbenzènesulfonates, les alkylnaphtalènesulfonates, les sulfates, les éthersulfates et les sulfonates d'alcools gras, les sels d'ammonium quaternaires, tels que le bromure de triméthylcétylammonium, le bromure de cétylpyridinium, les éthanolamides d'acides gras éventuellement oxyéthylénés, les alcools gras polyglycérolés, les alkylphénols polyoxyéthylénés ou polyglycérolés, ainsi que les alkylsulfates polyoxyéthylénés.

Ces agents tensio-actifs sont présents dans les compositions conformes à l'invention dans des proportions comprises entre 0,5 et 55 % en poids, et de préférence entre 2 et 50 % en poids par rapport au poids total de la composition.

Ces compositions peuvent également contenir des solvants organiques pour solubiliser les composants qui ne seraient pas suffisamment solubles dans l'eau. Parmi ces solvants, on peut citer à titre d'exemple, les alcanols inférieurs en C₁-C₄, tels que l'éthanol et l'isopropanol; le glycérol; les glycols ou éthers de glycols comme le 2-butoxyéthanol, l'éthylèneglycol, le propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants sont présents de préférence dans des proportions comprises entre 1 et 40 % en poids, et en particulier entre 5 et 30 % en poids par rapport au poids total de la composition.

Les agents épaississants que l'on peut ajouter dans les compositions conformes à l'invention peuvent être choisis parmi l'alginate de sodium, la gomme arabique, les polymères d'acide acrylique éventuellement réticulés, les dérivés de cellulose, les hétérobiopolysaccharides tels que la gomme de xanthane, on peut également utiliser des agents épaississants minéraux tels que la bentonite.

Ces agents épaississants sont présents de préférence dans des proportions comprises entre 0,1 et 5 %, et en particulier entre 0,2 et 3 % en poids par rapport au poids total de la composition.

Les agents antioxydants qui peuvent être présents dans les compositions sont choisis en particulier parmi le sulfite de sodium, l'acide thioglycolique, le bisulfite de sodium, l'acide déhydroascorbique, l'hydroquinone, la 2-méthylhydroquinone et l'acide homogentisique. Ces agents antioxydants sont présents dans la composition dans des proportions comprises entre 0,05 et 1,5 % en poids par rapport au poids total de la composition.

Le pH de ces compositions est compris entre 4 et 11. Il est ajusté à la valeur désirée à l'aide d'agents alcalinisants bien connus de l'état de la technique, tels que l'ammoniaque, les carbonates alcalins, les alcanolamines tels que les mono-, di- et triéthanolamines ainsi que leurs dérivés ou les hydroxydes de sodium et de potassium, ou d'agents acidifiants classiques, tels que les acides minéraux ou organiques, tels que les acides chlorhydrique, tartrique, citrique, phosphorique et sulfonique.

Ces compositions peuvent également contenir d'autres adjuvants cosmétiquement acceptables, tels que par exemple des agents de pénétration, des agents séquestrants, des parfums, des tampons, etc.

Les compositions conformes à l'invention peuvent se présenter sous des formes diverses, telles que sous forme de liquide, de crème, de gel ou sous toute autre forme appropriée pour réaliser une teinture des fibres kératiniques et notamment des cheveux humains. Ces compositions peuvent être conditionnées en flacons aérosols en présence d'un agent propulseur et former des mousses.

Les composes de formule (I) sont utilisés conformément à l'invention selon un procédé comprenant l'application sur les fibres kératiniques du composé de formule (I) en présence ou non d'un ou plusieurs coupleurs et sont révélés par un agent oxydant.

Les compositions tinctoriales conformes à l'invention contenant au moins un composé de formule (I) et éventuellement au moins un coupleur, sont utilisées suivant un procédé mettant en oeuvre la révélation par un agent oxydant.

Conformément à ce procédé on mélange, au moment de l'emploi, la composition tinctoriale décrite ci-dessus avec une solution oxydante en une quantité suffisante pour pouvoir développer une coloration, puis on applique le mélange obtenu sur les fibres kératiniques et en particulier les cheveux humains.

Le pH de la composition appliquée sur les cheveux varie de préférence entre 3 et 11. Il est ajusté à la valeur désirée à l'aide d'agents alcalinisants ou acidifiants bien connus de l'état de la technique, tels que décrits précédemment. La solution oxydante contient à titre d'agent oxydant, l'eau oxygéné, le peroxyde d'urée, des persels, tels que le persulfate d'ammonium, des peracides organiques et leurs sels ou des bromates de métaux alcalins. On utilise de préférence une solution d'eau oxygénée à 20 volumes.

Le mélange obtenu est appliqué sur les cheveux et on laisse poser pendant 10 à 40 minutes, de préférence 15 à 30 minutes, après quoi on les rince, les lave au shampooing, on les rince à nouveau et on les sèche.

Le composé de formule (I) définie ci-dessus, peut également être mis en oeuvre dans un procédé en plusieurs étapes, consistant dans l'une des étapes, à appliquer le composé de formule (I) et, dans une autre étape, à appliquer une composition tinctoriale contenant au moins un coupleur et/ou au moins un autre précurseur de colorant d'oxydation différent de ceux de formule (I).

L'agent oxydant peut être introduit, juste avant l'application, dans la composition appliquée dans le deuxième temps ou bien être appliqué sur les fibres kératiniques elles-mêmes, dans un troisième temps, les conditions de pose, de pH, de lavage et de séchage étant identiques à celles indiquées ci-dessus.

Les exemples qui suivent sont destinés à illustrer l'invention.

### EXEMPLES DE PREPARATION

### Préparation du 3-hydroxyméthyl p-aminophénol.

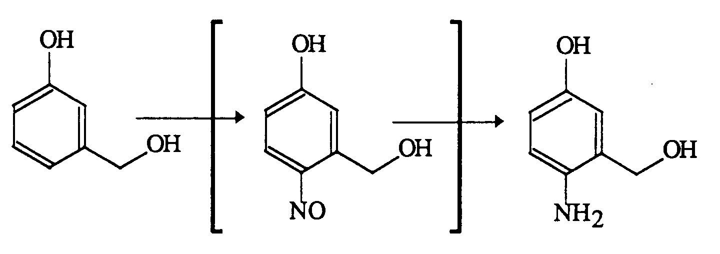

On mélange 31 g (0,25 mole) d'alcool métahydroxybenzylique, 10 g de soude, 17,5 g de nitrite de sodium et 380 g d'eau glacée.

On coule entre 0° et 5°C, 25 ml d'acide sulfurique concentré en 30 minutes; après 1 heure, on filtre le précipité beige. On ajoute le précipité humide dans une solution de 200 g d'hydrosulfite de sodium dans 600 ml de soude 2,5 N par spatulée à 80-90°C. Après 15 minutes, on refroidit, acidifie avec de l'acide sulfurique et traite avec 100 g de noir de carbone. Après filtration, on neutralise avec de l'ammoniaque. Après 2 heures à la température ambiante, on filtre le précipité, on reprend ce précipité par 4 fois 500 ml d'acétate d'éthyle. Les phases organiques sont séchées et évaporées. Le résidu est recristallisé dans l'éthanol à 95°.

On obtient des cristaux beiges avec un point de fusion de 171°C.

On obtient pour une formule C₇H₉NO₂ :

| | % | C | H | N | O |
|---|---|---|---|---|---|
| Calculé | | 60,93 | 6,47 | 10,07 | 23,02 |
| Trouvé | | 60,28 | 6,49 | 10,00 | 23,12 |

### Préparation du 3-cyanométhyl p-aminophénol.

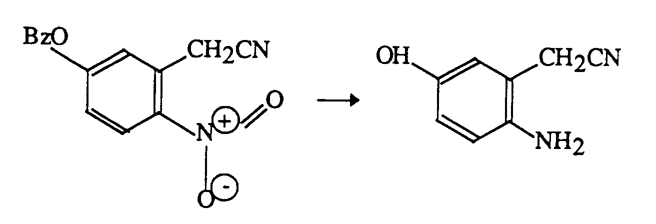

On porte au reflux une suspension de 5,4 g de 2-nitro 5-benzyloxyphénylacétonitrile, 5,4 ml de cyclohexène, 22 ml d'éthanol et 3 g de palladium à 10% sur charbon. Après 5 heures, on filtre la suspension à chaud, puis on évapore le filtrat. Le résidu est recristallisé de l'éthanol pour conduire à des cristaux beige foncé.

Point de fusion: 173°C.

| Analyse élémentaire pour C₈H₈N₂O | | | | | |
|---|---|---|---|---|---|
| | % | C | H | N | O |
| Calculé | | 64,85 | 5,44 | 18,91 | 10,80 |
| Trouvé | | 64,97 | 5,47 | 18,96 | 11,01 |

### Préparation du 3-(β-éthoxyéthyl)p-aminophénol.

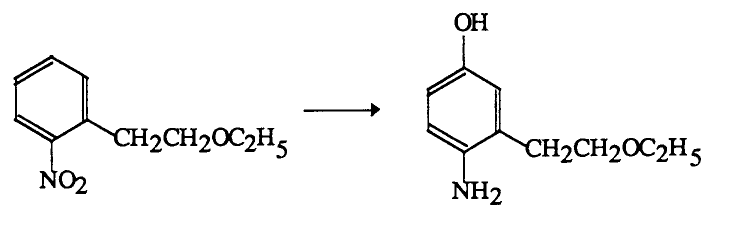

On ajoute à une température de 90°C, 58,5 g de 1-(β-éthoxyéthyl) 2-nitrobenzène (0,3 mole) à une solution de 100 ml d'acide sulfurique concentré dilué dans 1 litre d'eau distillée. On ajoute sous forte agitation, 17 g d'aluminium en poudre par spatulée. On maintient l'agitation pendant 40 minutes, puis on ajoute 70 g d'acide tartrique. On poursuit l'agitation pendant 15 minutes en maintenant la température à 90°C, puis on refroidit le milieu réactionnel et on filtre la suspension sur verre fritté. Le filtrat est neutralisé avec de l'ammoniaque à 20% à une température inférieure à 20°C. On obtient un précipité beige légèrement rosé. On reprend le précipité dans 0,5 l d'acétate d'éthyle au reflux, on filtre à chaud et on concentre à sec. On recristallise ensuite de l'acétate d'éthyle pour obtenir des cristaux beiges du produit attendu dont le point de fusion est de 102°C.

| Analyse élémentaire pour C₁₀H₁₅NO₂ | | | | | |
|---|---|---|---|---|---|
| | % | C | H | N | O |
| Calculé | | 66,27 | 8,34 | 7,73 | 18,66 |
| Trouvé | | 65,88 | 8,32 | 7,51 | 18,30 |

### Préparation du 3-(β-méthoxyéthyl)p-aminophénol.

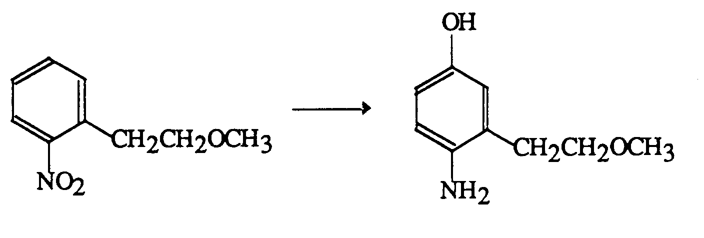

On procède comme pour l'exemple ci-dessus en partant du 1-(β-méthoxyéthyl)2-nitrobenzène (54,3 g, soit 0,3 mole) pour obtenir, après recristallisation de l'acétate d'éthyle, des cristaux blancs ayant un point de fusion de 82°C.

| Analyse élémentaire pour C₉H₁₃NO₂ | | | | | |
|---|---|---|---|---|---|
| | % | C | H | N | O |
| Calculé | | 64,67 | 7,78 | 8,38 | 19,16 |
| Trouvé | | 64,46 | 7,86 | 8,33 | 19,29 |

### EXEMPLES DE TEINTURE

### Teinture à pH basique

### EXEMPLES 1 à 6

On prépare la composition tinctoriale suivante :

| | |
|---|---|
| - Colorants | x g |
| - Alcool oléique glycérolé à 2 moles de glycérol | 5 g |
| - Alcool oléique glycérolé à 4 moles de glycérol | 5 g |
| - Acide oléique | 5 g |
| - Diéthanolamine oléique | 5 g |
| - Diéthanolamide oléique | 12 g |
| - Alcool éthylique | 10 g |
| - Ethoxy-2 éthanol | 12 g |
| - Métabisulfite de sodium en solution aqueuse à 35% de MA | 1,3 g |
| - 2-méthylhydroquinone | 0,17 g |
| - Ammoniaque à 20% de NH₃ | 10,2 g |
| - Eau qsp | 100 g |

Au moment de l'emploi, on mélange cette composition poids pour poids avec une solution d'eau oxygénée à 20 volumes (6% en poids) et dont le pH est de 3.

On applique ce mélange sur des cheveux permanentés ou non permanentés, gris à 90% de blancs, pendant 30 minutes, puis on rince les cheveux et on effectue un shampooing. Après séchage, les cheveux sont teints dans la nuance précisée au bas du tableau ci-après.

| EXEMPLE | 1 | 2 | 3 | 4 | 5 | 6 |
|---|---|---|---|---|---|---|
| 3-éthyl p-aminophénol | 0,685 g | 0,685 g | 0,685 g | | | |
| 3-hydroxyméthyl p-aminophénol | | | | 0,695 g | 0,695 g | 0,695 g |
| 2-méthyl 5-aminophénol | 0,615 g | | | 0,615 g | | |
| 2,4-diaminophénoxyéthanol | | 1,2 g | | | 1,2 g | |
| 6-hydroxyindole | | | 0,665 g | | | 0,665 g |
| pH du mélange appliqué sur les cheveux | 10,3 | 10 | 10,2 | 10,3 | 10,1 | 10,3 |

| **NUANCE OBTENUE** | | | | | | |
|---|---|---|---|---|---|---|
| Cheveux gris naturels à 90 % blancs | | Prune | | | Châtain rouge | |
| Cheveux gris permanentés à 90 % blancs | Châtain moyen cuivré | | Blond foncé | Cuivré foncé | | Blond cuivré foncé |

### EXEMPLES 7 à 16

On prépare la composition tinctoriale suivante :

| | |
|---|---|
| - Colorants | x g |
| - Alcool oléique polyglycérolé à 2 moles de glycérol | 4 g |
| - Alcool oléique polyglycérolé à 4 moles de glycérol | |
| (78% de MA) | 5,69 g MA |
| - Acide oléique | 3 g |
| - Amine oléique à 2 moles d'oxyde d'éthylène, vendue sous la dénomination "ETHOMEEN O12" par la Société AKZO | 7 g |
| - Laurylamino succinamate de diéthylaminopropyle, sel de sodium à 55% de MA | 3 g MA |
| - Alcool oléique | 5 g |
| - Diéthanolamide d'acide oléique | 12 g |
| - Propylèneglycol | 3,5 g |
| - Alcool éthylique | 7 g |
| - Dipropylèneglycol | 0,5 g |
| - Monométhyléther de propylèneglycol | 9 g |
| - Métabisulfite de sodium en solution aqueuse à 35% de MA | 0,455 g MA |
| - Acétate d'ammonium | 0,8 g |
| - Antioxydant, séquestrant qs | |
| - Parfum, conservateur qs | |
| - Ammoniaque à 20% de NH₃ | 10 g |
| - Eau déminéralisée qsp | 100 g |

Au moment de l'emploi, on mélange cette composition, poids pour poids, avec une solution d'eau oxygéné à 20 volumes (6% en poids) et dont le pH est de 3.

On applique ce mélange sur des cheveux permanentés ou non permanentés, gris à 90% de blancs, pendant 30 minutes, puis on rince les cheveux et on effectue un shampooing.

Après séchage, les cheveux sont teints dans la nuance précisée au bas du tableau ci-après.

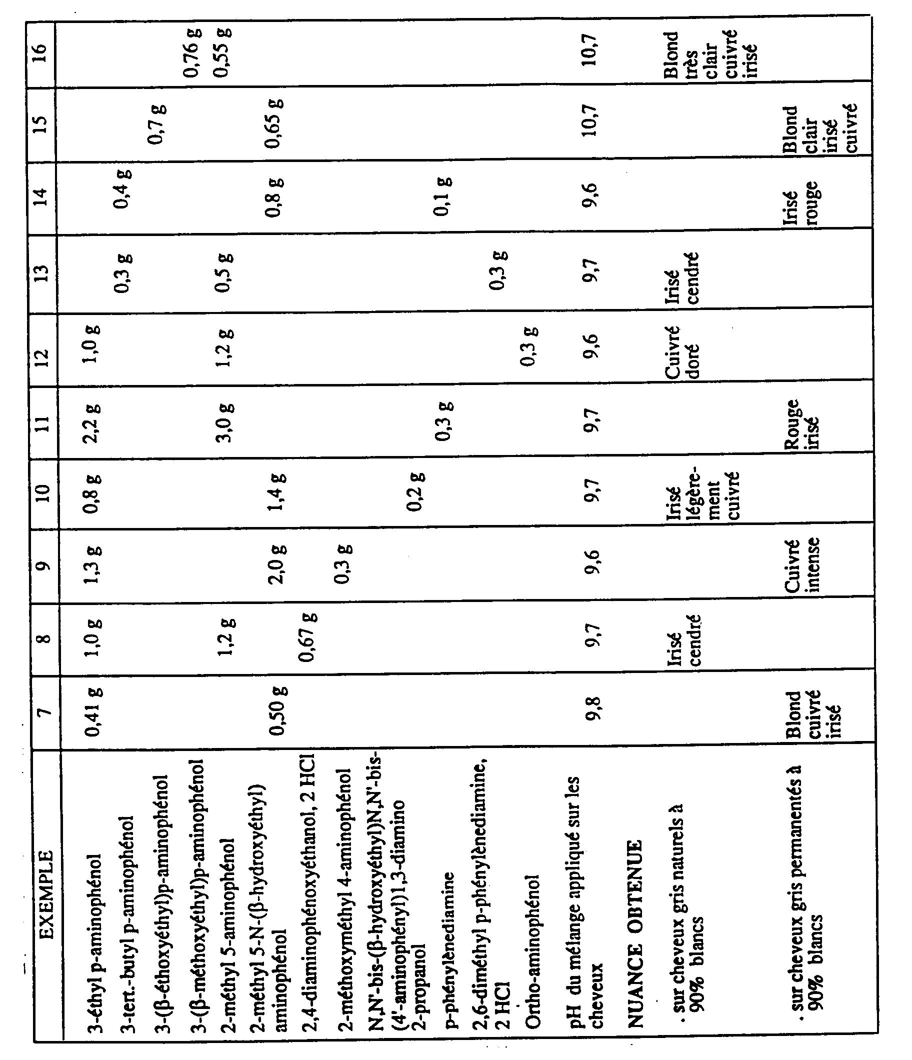

### EXEMPLE 17

On prépare la composition tinctoriale suivante :

| | |
|---|---|
| - 3-cyanométhyl p-aminophénol | 0,37 g |
| - 2-méthyl 5-N-(β-hydroxyéthyl)aminophénol | 0,41 g |
| - Alcool oléique polyglycérolé à 2 moles de glycérol | 4,5 g |
| - Alcool oléique polyglycérolé à 4 moles de glycérol | 4,5 g |
| - Amine oléique à 2 moles d'oxyde d'éthylène, vendue sous la dénomination "ETHOMEEN O12" par la Société AKZO | 4,5 g |
| - Diéthanolamide de coprah vendu sous la dénomination "COMPERLAN KD" par la Société HENKEL | 9 g |
| - Propylèneglycol | 4 g |
| - 2-butoxyéthanol | 8 g |
| - Ethanol à 96° | 6 g |
| - Sel pentasodique de l'acide diéthylènetriamine pentacétique, vendu sous la dénomination "MASQUOL DTPA" par la Société PROTEX | 2 g |
| - Hydroquinone | 0,15 g |
| - Solution aqueuse de métabisulfite de sodium à 35% de MA | 1,3 g |
| - Ammoniaque à 20% de NH₃ | 10 g |
| - Eau qsp | 100 g |

Au moment de l'emploi, on ajoute 100 g d'eau oxygéné à 20 volumes (6% en poids) et dont le pH est de 3. Le pH de ce mélange est de 10. Il est appliqué pendant 25 minutes et à une température de 30°C sur des cheveux gris naturels à 90% de blancs. Ce mélange leur confère, après shampooing et rinçage, une coloration beige cendré.

### Teinture à pH acide

### EXEMPLE 18

On prépare la composition tinctoriale suivante :

| | |
|---|---|
| - 3-éthyl p-aminophénol | 0,41 g |
| - 2-hydroxy 4-N-(β-hydroxyéthyl)aminoanisole | 0,768 g |
| - Alcool oléique polyglycérolé à 2 moles de glycérol | 4 g |
| - Alcool oléique polyglycérolé à 4 moles de glycérol (78% de MA) | 5,69 g MA |
| - Acide oléique | 3 g |
| - Amine oléique à 2 moles d'oxyde d'éthylène, vendue sous la dénomination "ETHOMEEN O12" par la Société AKZO | 7 g |
| - Laurylaminosuccinamate de diéthylaminopropyle, sel de sodium à 55% de MA | 3 g MA |
| - Alcool oléique | 5 g |
| - Diéthanolamide d'acide oléique | 12 g |
| - Propylèneglycol | 3,5 g |
| - Alcool éthylique | 7 g |
| - Dipropylèneglycol | 0,5 g |
| - Monométhyléther de propylèneglycol | 9 g |
| - Métabisulfite de sodium en solution aqueuse à 35% de MA | 0,455 g MA |
| - Acétate d'ammonium | 0,8 g |
| - Antioxydant, séquestrant qs | |
| - Parfum, conservateur qs | |
| - Monoéthanolamine qsp pH=9,8 | |
| - Eau déminéralisée qsp | 100 g |

Au moment de l'emploi, on mélange cette composition, poids pour poids, avec une solution d'eau oxygéné à 20 volumes (6% en poids) et dont le pH est ajusté entre 1 et 1,5 par 2,5 g d'acide orthophosphorique pour 100 g d'eau oxygénée.

Le pH du mélange est de 6,5.

Ce mélange est appliqué sur des cheveux gris à 90% de blancs, permanentés, pendant 30 minutes.

Les cheveux sont ensuite rincés, lavés avec un shampooing, puis séchés. Ils sont teints en blond très clair légèrement doré.

### EXEMPLE 19

On prépare la composition tinctoriale suivante :

| | |
|---|---|
| - 3-hydroxyméthyl p-aminophénol | 0,9 g |
| - 2-méthyl 5-aminophénol | 1,2 g |
| - p-phénylènediamine | 0,3 g |
| - Alcool oléique polyglycérolé à 2 moles de glycérol | 4 g |
| - Alcool oléique polyglycérolé à 4 moles de glycérol (78% de MA) | 5,69 g MA |
| - Acide oléique | 3 g |
| - Amine oléique à 2 moles d'oxyde d'éthylène, vendue sous la dénomination "ETHOMEEN O12" par la Société AKZO | 7 g |
| - Laurylaminosuccinamate de diéthylaminopropyle, sel de sodium à 55% de MA | 3 g MA |
| - Alcool oléique | 5 g |
| - Diéthanolamide d'acide oléique | 12 g |
| - Propylèneglycol | 3,5 g |
| - Alcool éthylique | 7 g |
| - Dipropylèneglycol | 0,5 g |
| - Monométhyléther de propylèneglycol | 9 g |
| - Métabisulfite de sodium en solution aqueuse à 35% de MA | 0,455 g MA |
| - Acétate d'ammonium | 0,8 g |
| - Antioxydant, séquestrant qs | |
| - Parfum, conservateur qs | |
| - Monoéthanolamine qsp pH=9,8 | |
| - Eau déminéralisée qsp | 100 g |

Au moment de l'emploi, on mélange cette composition, poids pour poids, avec une solution d'eau oxygénée à 20 volumes (6% en poids) et dont le pH est ajusté entre 1 et 1,5 par 2,5 g d'acide orthophosphorique pour 100 g d'eau oxygénée.

Le pH du mélange est de 6,8.

Ce mélange est appliqué sur des cheveux gris naturels à 90% de blancs pendant 30 minutes.

Les cheveux sont ensuite rincés, lavés avec un shampooing, puis séchés. Ils sont teints en blond irisé acajou.

## Revendications

1. Utilisation pour la teinture de fibres kératiniques, et en particulier de cheveux humains, d'un para-aminophénol 3-substitué de formule : 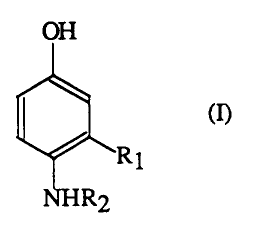 dans laquelle R₁ représente un radical alkyle en C₁-C₄, un radical alcényle en C₂-C₄, un radical monohydroxyalkyle en C₁-C₆, polyhydroxyalkyle en C₂-C₆, alcoxyalkyle en C₂-C₆, nitrile, cyanoalkyle en C₁-C₄, halogénoalkyle en C₁-C₄ et de préférence fluoroalkyle en C₁-C₄, aminoalkyle de formule : 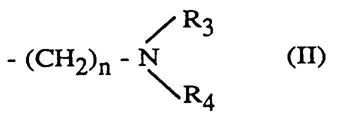 dans laquelle :
n est un nombre entier compris entre 1 et 6 inclus;
R₃ et R₄ identiques ou différents, représentent un atome d'hydrogène ou un radical alkyle en C₁-C₄, hydroxyalkyle en C₁-C₄, acyle en C₁-C₆;
R₂ représente un atome d'hydrogène, un radical alkyle en C₁-C₆, un radical monohydroxyalkyle en C₁-C₆, polyhydroxyalkyle en C₂-C₆, sous réserve que :
lorsque R₂ est hydrogène, R₁ ne représente pas méthyle ou trifluorométhyle,
ou de l'un de ses sels d'addition avec un acide.

2. Utilisation selon la revendication 1, d'un para-aminophénol 3-substitué de formule (I) choisi parmi le 3-éthyl p-aminophénol, le 3-hydroxyméthyl p-aminophénol, le 3-cyanométhyl p-aminophénol, le 3-tert.-butyl p-aminophénol, le 3-(β-méthoxyéthyl)p-aminophénol et le 3-(β-éthoxyéthyl)p-aminophénol.

3. Utilisation selon la revendication 1 ou 2, d'un para-aminophénol 3-substitué de formule (I) en présence d'un ou plusieurs coupleurs choisis parmi les métadiphénols, les métaaminophénols, les métaphénylènediamines, les métaacylaminophénols, les métauréidophénols, les métacarbalcoxyaminophénols, l'α-naphtol, les dérivés indoliques, les composés β-cétoniques et les pyrazolones.

4. Composition tinctoriale pour fibres kératiniques, et en particulier pour cheveux humains, caractérisée par le fait qu'elle comprend, dans un milieu aqueux approprié pour la teinture, au moins un para-aminophénol 3-substitué de formule : 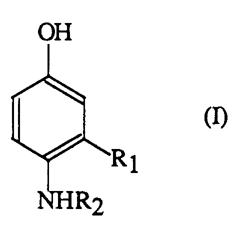 dans laquelle R₁ représente un radical alkyle en C₁-C₄, un radical alcényle en C₂-C₄, un radical monohydroxyalkyle en C₁-C₆, polyhydroxyalkyle en C₂-C₆, alcoxyalkyle en C₂-C₆, nitrile, cyanoalkyle en C₁-C₄, halogénoalkyle en C₁-C₄ et de préférence fluoroalkyle en C₁-C₄, aminoalkyle de formule : 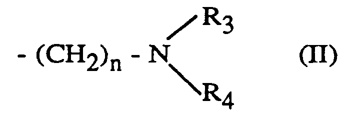 dans laquelle :
n est un nombre entier compris entre 1 et 6 inclus;
R₃ et R₄ identiques ou différents, représentent un atome d'hydrogène ou un radical alkyle en C₁-C₄, hydroxyalkyle en C₁-C₄, acyle en C₁-C₆;
R₂ représente un atome d'hydrogène, un radical alkyle en C₁-C₆, un radical monohydroxyalkyle en C₁-C₆, polyhydroxyalkyle en C₂-C₆, sous réserve que :
lorsque R₂ est hydrogène, R₁ ne représente pas méthyle ou trifluorométhyle,
ou de l'un de ses sels d'addition avec un acide.

5. Composition tinctoriale selon la revendication 4, caractérisée par le fait que le para-aminophénol 3-substitué de formule (I) est choisi parmi le 3-éthyl p-aminophénol, le 3-hydroxyméthyl p-aminophénol, le 3-cyanométhyl p-aminophénol, le 3-tert.-butyl p-aminophénol, le 3-(β-méthoxyéthyl)p-aminophénol, le 3-(β-éthoxyéthyl)p-aminophénol, ou l'un de leurs sels.

6. Composition tinctoriale selon la revendication 4 ou 5, caractérisée par le fait qu'elle contient en outre au moins un coupleur choisi parmi les métadiphénols, les métaaminophénols, les métaphénylènediamines₁ les métaacylaminophénols, les métauréidophénols, les métacarbalcoxyaminophénols, l'α-naphtol, les dérivés indoliques, les composés β-cétoniques et les pyrazolones.

7. Composition tinctoriale selon l'une quelconque des revendications 4 à 6, caractérisée par le fait qu'elle contient au moins un autre précurseur de colorant d'oxydation de type para ou ortho choisi parmi les paraphénylènediamines, les para-aminophénols différents de ceux de formule (I), les précurseurs hétérocycliques para dérivés de la pyridine ou de la pyrimidine, les orthoaminophénols et les bis-phénylalkylènediamines.

8. Composition tinctoriale selon l'une quelconque des revendications 4 à 7, caractérisée par le fait qu'elle contient le 3-éthyl p-aminophénol ou l'un de ses sels à titre de précurseur de colorant d'oxydation, au moins un coupleur choisi parmi le 2-méthyl 5-amino-phénol et le 2-méthyl 5-N-(β-hydroxyéthyl)aminophénol ou l'un de leurs sels, et au moins une métaphénylènediamine ou l'un de ses sels, à titre de coupleur additionnel.

9. Composition tinctoriale selon l'une quelconque des revendications 4 à 7, caractérisée par le fait qu'elle contient le 3-éthyl p-aminophénol ou l'un de ses sels à titre de précurseur de colorant d'oxydation, au moins un coupleur choisi parmi le 2-méthyl 5-aminophénol et le 2-méthyl 5-N-(β-hydroxyéthyl)aminophénol ou l'un de leurs sels et un précurseur de colorant d'oxydation additionnel choisi parmi les orthoaminophénols, les paraphénylènediamines, les bis-phénylalkylènediamines et les para-aminophénols différents de ceux de formule (I).

10. Composition tinctoriale selon l'une quelconque des revendications 4 à 9, caractérisée par le fait qu'elle contient de 0,05 à 3,5% en poids, par rapport au poids total de la composition, d'au moins un para-aminophénol 3-substitué de formule (I).

11. Composition tinctoriale selon l'une quelconque des revendications 4 à 10, caractérisée par le fait qu'elle contient en tout de 0,3 à 7% en poids, par rapport au poids total de la composition, de précurseurs de colorants d'oxydation de type para et/ou ortho et de coupleurs.

12. Composition tinctoriale selon l'une quelconque des revendications 4 à 11, caracterisée par le fait qu'elle contient en outre au moins un adjuvant choisi parmi les agents tensio-actifs anioniques, cationiques, non-ioniques, amphotères ou leurs mélanges, en des proportions comprises entre 0,5 et 55% en poids, les solvants organiques en des concentrations comprises entre 1 et 40% en poids, les agents épaississants en des proportions comprises entre 0,1 et 5% en poids, les agents antioxydants en des proportions comprises entre 0,05 et 1,5% en poids, les colorants directs, les agents de pénétration, les agents séquestrants, les parfums et les tampons, les pourcentages en poids étant donnés par rapport au poids total de la composition.

13. Composition tinctoriale selon l'une quelconque des revendications 4 à 12, caractérisée par le fait qu'elle se présente sous forme de liquide, crème, gel ou mousse et qu'elle possède un pH compris entre 4 et 11.

14. Procédé de teinture d'oxydation des fibres kératiniques et en particulier des cheveux humains, caractérisé par le fait qu'on applique sur les fibres kératiniques au moins un para-aminophénol 3-substitué de formule (I) suivante : 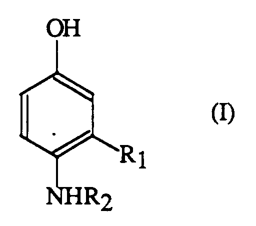 dans laquelle R₁ représente un radical alkyle en C₁-C₄, un radical alcényle en C₂-C₄, un radical monohydroxyalkyle en C₁-C₆, polyhydroxyalkyle en C₂-C₆, alcoxyalkyle en C₂-C₆, nitrile, cyanoalkyle en C₁-C₄, halogénoalkyle en C₁-C₄ et de préférence fluoroalkyle en C₁-C₄, aminoalkyle de formule : 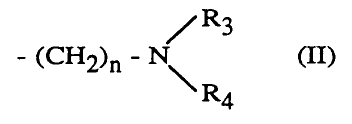 dans laquelle :
n est un nombre entier compris entre 1 et 6 inclus;
R₃ et R₄ identiques ou différents, représentent un atome d'hydrogène ou un radical alkyle en C₁-C₄, hydroxyalkyle en C₁-C₄, acyle en C₁-C₆;
R₂ représente un atome d'hydrogène, un radical alkyle en C₁-C₆, un radical monohydroxyalkyle en C₁-C₆, polyhydroxyalkyle en C₂-C₆, sous réserve que :
lorsque R₂ est hydrogène, R₁ ne représente pas méthyle ou trifluorométhyle,
ou l'un de ses sels d'addition avec un acide, en présence ou non d'un ou plusieurs coupleurs, puis on développe la couleur à l'aide d'un agent oxydant.

15. Procédé selon la revendication 14, caractérisé par le fait qu'on mélange, au moment de l'emploi, une composition tinctoriale contenant, dans un milieu aqueux approprié pour la teinture, au moins un para-aminophénol 3-substitué de formule (I) et éventuellement au moins un coupleur, avec une solution oxydante en une quantité suffisante pour développer la coloration, le pH de la composition résultante variant entre 3 et 11, et on applique le mélange ainsi obtenu sur les fibres kératiniques, et en particulier les cheveux humains, on laisse poser pendant 10 à 40 minutes, puis on rince, on lave au shampooing, on rince à nouveau et on sèche.

16. Procédé de teinture selon la revendication 14, caractérisé par le fait que dans un premier temps, on applique au moins un paraaminophénol 3-substitué de formule (I) sur les fibres kératiniques, dans un deuxième temps, on applique une composition tinctoriale contenant au moins un coupleur et/ou au moins un autre précurseur de colorant d'oxydation différent de ceux de formule (I), et on développe la coloration à l'aide d'un agent oxydant présent dans la composition appliquée dans le deuxième temps, ou bien appliqué sur les fibres kératiniques dans un troisième temps, on laisse poser pendant 10 à 40 minutes, on rince, on lave au shampooing, on rince à nouveau et on sèche.

17. Para-aminophénol 3-substitué de formule : 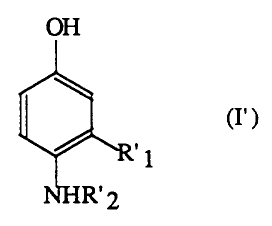 dans laquelle R'₁ représente un radical alkyle en C₁-C₄, un radical alcényle en C₂-C₄, un radical monohydroxyalkyle en C₁-C₆, polyhydroxyalkyle en C₂-C₆, alcoxyalkyle en C₂-C₆, nitrile, cyanoalkyle en C₁-C₄, halogénoalkyle en C₁-C₄ et de préférence fluoroalkyle en C₁-C₄, aminoalkyle de formule : 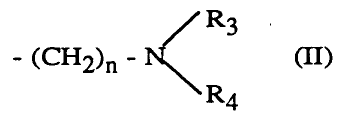 dans laquelle :
n est un nombre entier compris entre 1 et 6 inclus;
R₃ et R₄ identiques ou différents, représentent un atome d'hydrogène ou un radical alkyle en C₁-C₄, hydroxyalkyle en C₁-C₄, acyle en C₁-C₆;
R'₂ représente un atome d'hydrogène, un radical alkyle en C₁-C₆, un radical monohydroxyalkyle en C₁-C₆, polyhydroxyalkyle en C₂-C₆,
sous réserve que :
- lorsque R'₂ désigne un atome d'hydrogène, R'₁ ne représente pas un radical alkyle, vinyle, dichlorométhyle ou trifluorométhyle;
- lorsque R'₁ désigne le radical méthyle, R'₂ ne peut désigner le radical éthyle,
ou l'un de ses sels d'addition avec un acide.

18. Para-aminophénol 3-substitué de formule (I') selon la revendication 17, caractérisé par le fait qu'il est choisi parmi le 3-hydroxyméthyl p-aminophénol, le 3-cyanométhyl p-aminophénol, le 3-(β-méthoxyéthyl)p-aminophénol, le 3-(β-éthoxyéthyl)p-aminophénol, ou l'un de leurs sels d'addition avec un acide.

## Claims

1. Use in dyeing keratinous fibres, in particular human hair, of a 3-substituted para-aminophenol with formula: 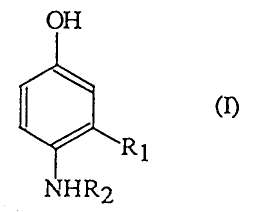 where R₁ represents a C₁-C₄ alkyl radical, a C₂-C₄ alkenyl radical, a C₁-C₆ monohydroxyalkyl, a C₂-C₆ polyhydroxyalkyl radical, a C₂-C₆ alkoxyalkyl radical, a nitrile radical, a C₁-C₄ cyanoalkyl radical, a C₁-C₄ halogenoalkyl radical, preferably a C₁-C₄ fluoroalkyl radical, an aminoalkyl radical with formula: 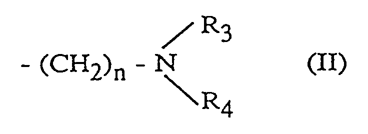 where:
n is a whole number from 1 to 6 inclusive;
R₃ and R₄, which may be identical or different, represent a hydrogen atom or a C₁-C₄ alkyl radical, a C₁-C₄ hydroxyalkyl radical or a C₁-C₆ acyl radical;
R₂ represents a hydrogen atom, a C₁-C₆ alkyl radical, a C₁-C₆ monohydroxyalkyl radical or a C₂-C₆ polyhydroxyalkyl radical, provided that:
when R₂ is hydrogen, R₁ does not represent methyl or trifluoromethyl,
or an addition salt thereof with an acid.

2. Use of a 3-substituted para-aminophenol with formula (I) according to claim 1, selected from 3-ethyl p-aminophenol, 3-hydroxymethyl p-aminophenol, 3-cyanomethyl p-aminophenol, 3-tert-butyl p-aminophenol, 3-(β-methoxyethyl)p-aminophenol and 3-(β-ethoxyethyl) p-aminophenol.

3. Use of a 3-substituted para-aminophenol with formula (I) according to claim 1 or claim 2, in the presence of one or more couplers selected from metadiphenols, metaaminophenols, metaphenylenediamines, metaacylaminophenols, metaureidophenols, metacarbalkoxyaminophenols, α-naphthol, indole derivatives, β-ketone compounds and pyrazoles.

4. Dye composition for keratinous fibres, in particular human hair, characterised in that it contains, in an appropriate aqueous dye medium, at least one 3-substituted para-aminophenol with formula: 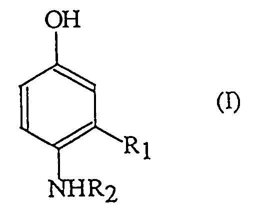 where R₁ represents a C₁-C₄ alkyl radical, a C₂-C₄ alkenyl radical, a C₁-C₆ monohydroxyalkyl, a C₂-C₆ polyhydroxyalkyl radical, a C₂-C₆ alkoxyalkyl radical, a nitrile radical, a C₁-C₄ cyanoalkyl radical, a C₁-C₄ halogenoalkyl radical, preferably a C₁-C₄ fluoroalkyl radical, an aminoalkyl radical with formula: 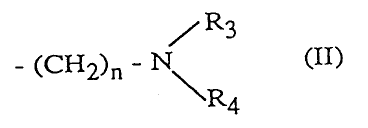 where:
n is a whole number from 1 to 6 inclusive;
R₃ and R₄, which may be identical or different, represent a hydrogen atom or a C₁-C₄ alkyl radical, a C₁-C₄ hydroxyalkyl radical or a C₁-C₆ acyl radical;
R₂ represents a hydrogen atom, a C₁-C₆ alkyl radical, a C₁-C₆ monohydroxyalkyl radical or a C₂-C₆ polyhydroxyalkyl radical, provided that:
when R₂ is hydrogen, R₁ does not represent methyl or trifluoromethyl,
or an addition salt thereof with an acid.

5. Dye composition according to claim 4 characterised in that the 3-substituted para-aminophenol with formula (I) is selected from 3-ethyl p-aminophenol, 3-hydroxymethyl p-aminophenol, 3-cyanomethyl p-aminophenol, 3-tert-butyl p-aminophenol, 3-(β-methoxyethyl) p-aminophenol, 3-(β-ethoxyethyl) p-aminophenol or a salt thereof.

6. Dye composition according to claim 4 or claim 5 characterised in that it further contains at least one coupler selected from metadiphenols, metaaminophenols, metaphenylenediamines, metaacylaminophenols, metaureidophenols, metacarbalkoxyaminophenols, α-naphthol, indole derivatives, β-ketone compounds and pyrazoles.

7. Dye composition according to any one of claims 4 to 6 characterised in that it contains at least one para or ortho type oxidation dye precursor selected from paraphenylenediamines, para-aminophenols other than those with formula (I), para heterocyclic derivatives of pyridine or pyrimidine, orthoaminophenols and bis-phenylalkylenediamines.

8. Dye composition according to any one of claims 4 to 7 characterised in that it contains 3-ethyl p-aminophenol or a salt thereof as an oxidation dye precursor, at least one coupler selected from 2-methyl 5-aminophenol and 2-methyl 5-N-(β-hydroxyethyl) aminophenol or a salt thereof and at least one metaphenylenediamine or a salt thereof to act as an additional coupler.

9. Dye composition according to any one of claims 4 to 7 characterised in that it contains 3-ethyl p-aminophenol or a salt thereof as an oxidation dye precursor, at least one coupler selected from 2-methyl 5-aminophenol and 2-methyl 5-N-(β-hydroxyethyl) aminophenol or a salt thereof and an additional oxidation dye precursor selected from orthoaminophenols, paraphenylenediamines, bis-phenylalkylenediamines and para-aminophenols other than those with formula (I).

10. Dye composition according to any one of claims 4 to 9 characterised in that it contains 0.05% to 3.5% by weight with respect to the total composition weight of at least one 3-substituted para-aminophenol with formula (I).

11. Dye composition according to any one of claims 4 to 9 characterised in that it contains a total of 0.3% to 7% by weight with respect to the total composition weight of ortho and/or para type oxidation dye precursors and couplers.

12. Dye composition according to any one of claims 4 to 11 characterised in that it further contains at least one additive selected from anionic, cationic, non-ionic and amphoteric surfactants or mixtures thereof, in proportions of between 0.5% and 55% by weight, organic solvents in concentrations of between 1% and 40% by weight, thickening agents in proportions of between 0.1% and 5% by weight, antioxidising agents in proportions of between 0.05% and 1.5% by weight, direct dyes, penetrating agents, sequestrating agents, perfumes and buffers, the percentages by weight being with respect to the total composition weight.

13. Dye composition according to any one of claims 4 to 12 characterised in that it is in the form of a liquid, cream, gel or mousse and in that it has a pH of between 4 and 11.

14. A method of oxidation dyeing keratinous fibres, in particular human hair, characterised in that at least one 3-substituted para-aminophenol with formula: 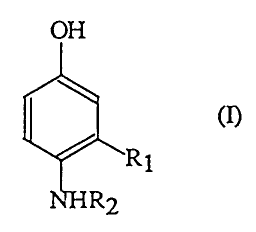 is applied to the hair,
in which formula R₁ represents a C₁-C₄ alkyl radical, a C₂-C₄ alkenyl radical, a C₁-C₆ monohydroxyalkyl, a C₂-C₆ polyhydroxyalkyl radical, a C₂-C₆ alkoxyalkyl radical, a nitrile radical, a C₁-C₄ cyanoalkyl radical, a C₁-C₄ halogenoalkyl radical, preferably a C₁-C₄ fluoroalkyl radical, an aminoalkyl radical with formula: 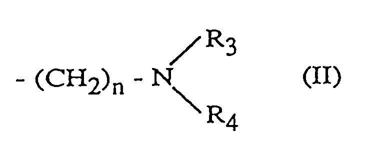 where:
n is a whole number from 1 to 6 inclusive;
R₃ and R₄, which may be identical or different, represent a hydrogen atom or a C₁-C₄ alkyl radical, a C₁-C₄ hydroxyalkyl radical or a C₁-C₆ acyl radical;
R₂ represents a hydrogen atom, a C₁-C₆ alkyl radical, a C₁-C₆ monohydroxyalkyl radical or a C₂-C₆ polyhydroxyalkyl radical, provided that:
when R₂ is hydrogen, R₁ does not represent methyl or trifluoromethyl,
or an addition salt thereof with an acid, optionally in the presence of one or more couplers, after which the colour is developed using an oxidising agent.

15. Method according to claim 14 characterised in that a dye composition containing at least one 3-substituted para-aminophenol with formula (I) and optionally at least one coupler in an appropriate aqueous dye medium is mixed just before use with a sufficient quantity of oxidising solution to develop the colour, the pH of the resulting composition varying from 3 to 11, and the mixture thus produced is applied to the keratinous fibres, in particular human hair, left for 10 to 40 minutes and then rinsed out, after which the hair is shampooed, rinsed again and dried.

16. Method according to claim 14 characterised in that at lest one 3-substituted para-aminophenol with formula (I) is applied in a first step to the keratinous fibres, and in a second step a dye composition containing at least one coupler and/or at least one other oxidation dye precursor other than that with formula (I) is applied, the colour is developed using an oxidising agent present in the composition applied in the second step, or applied to the keratinous fibres in a third step, the composition is left for 10 to 40 minutes and then rinsed out, after which the hair is shampooed, rinsed again and dried.

17. 3-substituted para-aminophenol with formula: 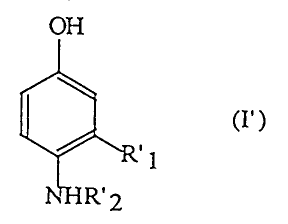 where R'₁ represents a C₁-C₄ alkyl radical, a C₂-C₄ alkenyl radical, a C₁-C₆ monohydroxyalkyl, a C₂-C₆ polyhydroxyalkyl radical, a C₂-C₆ alkoxyalkyl radical, a nitrile radical, a C₁-C₄ cyanoalkyl radical, a C₁-C₄ halogenoalkyl radical, preferably a C₁-C₄ fluoroalkyl radical, an aminoalkyl radical with formula: 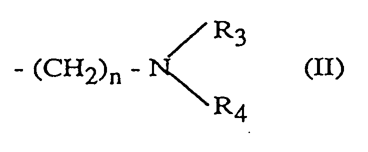 where:
n is a whole number from 1 to 6 inclusive;
R₃ and R₄, which may be identical or different, represent a hydrogen atom or a C₁-C₄ alkyl radical, a C₁-C₄ hydroxyalkyl radical or a C₁-C₆ acyl radical;
R'₂ represents a hydrogen atom, a C₁-C₆ alkyl radical, a C₁-C₆ monohydroxyalkyl radical or a C₂-C₆ polyhydroxyalkyl radical,
provided that:
- when R'₂ represents a hydrogen atom, R'₁ does not represent an alkyl, vinyl, dichloromethyl or trifluoromethyl radical;
- when R'₁ represents a methyl radical, R'₂ does not represent an ethyl radical,
or an addition salt thereof with an acid.

18. 3-substituted para-aminophenol with formula (I') according to claim 17 characterised in that it is selected from 3-hydroxymethyl p-aminophenol, 3-cyanomethyl p-aminophenol, 3-(β-methoxyethyl) p-aminophenol and 3-(β-ethoxyethyl) p-aminophenol, or an addition salt thereof with an acid.

## Patentansprüche

1. Zur Färbung keratinischer Fasern und insbesondere menschlicher Haare vorgesehene Verwendung eines 3-substituierten p-Aminophenols der Formel: 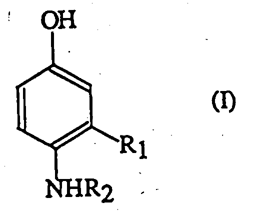 worin R₁ einen C₁₋₄-Alkyl-, C₂₋₄-Alkenyl-, c₁₋₆-Monohydroxyalkyl-, C₂₋₆-Polyhydroxyalkyl-, C₂₋₆-Alkoxyalkyl-, Nitril-, C₁₋₄-Cyanoalkyl-, C₁₋₄-Halogenalkyl- und vorzugsweise einen C₁₋₄-Fluoralkyl- sowie einen Aminoalkylrest der Formel darstellt: 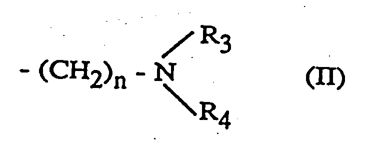 worin gilt:
n ist eine ganze Zahl von 1 bis 6;
R₃ und R₄ stellen, gleich oder verschieden, ein Wasserstoffatom oder einen C₁₋₄-Alkyl-, C₁₋₄-Hydroxyalkyl- und einen C₁₋₆-Acylrest dar;
und worin R₂ ein Wasserstoffatom, einen C₁₋₆-Alkyl-, C₁₋₆-Monohydroxyalkyl- oder einen C₂₋₆-Polyhydroxyalkylrest darstellt, mit der Maßgabe, daß:
wenn R₂ Wasserstoff ist, R₁ keinen Methyl- oder Trifluormethylrest darstellt,
oder eines seiner Additionssalze mit einer Säure.

2. Verwendung gemäß Anspruch 1 eines 3-substituierten p-Aminophenols der Formel (I), ausgewählt aus 3-Ethyl-p-aminophenol, 3-Hydroxymethyl-p-aminophenol, 3-Cyanomethyl-p-aminophenol, 3-t-Butyl-p-aminophenol, 3-(β-Methoxyethyl)-p-aminophenol und aus 3-(β-Ethoxyethyl)-p-aminophenol.

3. Verwendung gemäß Anspruch 1 oder 2 eines 3-substituierten p-Aminophenols der Formel (I) in Gegenwart eines oder mehrerer Kuppler, ausgewählt aus m-Diphenolen, m-Aminophenolen, m-Phenylendiaminen, m-Acylaminophenolen, m-Ureidophenolen, m-Carbalkoxyaminophenolen, α-Naphthol, Indolderivaten, β-Ketoverbindungen und aus Pyrazolonen.

4. Färbezusammensetzung für ketatinische Fasern und insbesondere für menschliche Haare,
dadurch **gekennzeichnet**, daß
sie, in einem zur Färbung geeigneten Medium, mindestens ein 3-substituiertes p-Aminophenol der Formel: 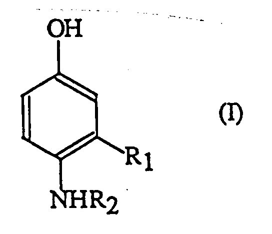 worin R₁ einen C₁₋₄-Alkyl-, C₂₋₄-Alkenyl-, C₁₋₆-Monohydroxyalkyl-, C₂₋₄-Polyhydroxyalkyl-, C₂₋₆-Alkoxyalkyl-, Nitril-, C₁₋₄-Cyanoalkyl-, C₁₋₄-Halogenalkyl- und vorzugsweise einen C₁₋₄-Fluoralkyl- sowie einen Aminoalkylrest der Formel darstellt: 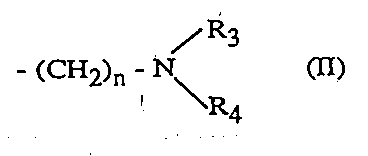 worin gilt:
n ist eine ganze Zahl von 1 bis 6;
R₃ und R₄ stellen, gleich oder verschieden, ein Wasserstoffatom oder einen C₁₋₄-Alkyl-, C₁₋₄-Hydroxyalkyl- und einen C₁₋₆-Acylrest dar;
und worin R₂ ein Wasserstoffatom, einen C₁₋₆-Alkyl-, C₁₋₆-Monohydroxyalkyl- oder einen C₂₋₆-Polyhydroxyalkylrest darstellt, mit der Maßgabe, daß:
wenn R₂ Wasserstoff ist, R₁ keinen Methyl- oder Trifluormethylrest darstellt,
oder eines seiner Additionssalze mit einer Säure enthält.

5. Färbezusammensetzung gemäß Anspruch 4,
dadurch **gekennzeichnet**, daß
das 3-substituierte p-Aminophenol der Formel (I) aus 3-Ethyl-p-aminophenol, 3-Hydroxymethl-p-aminophenol, 3-Cyanomethyl-p-aminophenol, 3-t-Butyl-p-aminophenol, 3-(β-Methoxyethyl)-p-aminophenol, 3-(β-Ethoxyethyl)-p-aminophenol oder aus einem seiner Salze ausgewählt ist.

6. Färbezusammensetzung gemäß Anspruch 4 oder 5,
dadurch **gekennzeichnet**, daß
sie ausserdem mindestens einen Kuppler enthält, ausgewählt aus m-Diphenolen, m-Aminophenolen, m-Phenylendiaminen, m-Acylaminophenolen, m-Ureidophenolen, m-Carbalkoxyphenolen, α-Naphthol, Indolderivaten, β-Ketoverbindungen und aus Pyrazolonen.

7. Färbezusammensetzung gemäß einem der Ansprüche 4 bis 6,
dadurch **gekennzeichnet**, daß
sie mindestens eine weitere Oxidationsfarbstoff-Vorstufenverbindung vom p- oder o-Typ enthält, ausgewählt aus p-Phenylendiaminen, p-Aminophenolen, die sich von denen der Formel (I) unterscheiden, aus heterozyklischen Vorstufenverbindungen von p-Derivaten des Pyridins oder Pyrimidins, aus o-Aminophenolen und aus Bisphenylalkylendiaminen.

8. Färbezusammensetzung gemäß einem der Ansprüche 4 bis 7,
dadurch **gekennzeichnet**, daß
sie 3-Ethyl-p-aminophenol oder eines seiner Salze als Oxidationsfarbstoff-Vorstufenverbindung, mindestens einen Kuppler, ausgewählt aus 2-Methyl-5-aminophenol und 2-Methyl-5-N-(β-hydroxyethyl)aminophenol oder aus einem von deren Salzen, und mindestens ein m-Phenylendiamin oder eines seiner Salze als zusätzlichen Kuppler enthält.

9. Färbezusammensetzung gemäß einem der Ansprüche 4 bis 7,
dadurch **gekennzeichnet**, daß
sie 3-Ethyl-p-aminophenol oder eines seiner Salze als Oxidationsfarbstoff-Vorstufenverbindung, mindestens einen Kuppler, ausgewählt aus 2-Methyl-5-aminophenol und 2-Methyl-5-N-(β-hydroxyethyl)aminophenol oder aus einem von deren Salzen , und eine zusätzliche Oxidationsfarbstoff-Vorstufenverbindung enthält, ausgewählt aus o-Aminophenolen, p-Phenylendiaminen, Bisphenylalkylendiaminen und aus p-Aminophenolen, die sich von denen der Formel (I) unterscheiden.

10. Färbezusammensetzung gemäß einem der Ansprüche 4 bis 9,
dadurch **gekennzeichnet**, daß
sie 0,05 bis 3,5 Gew.%, bezogen auf das Gesamtgewicht der Zusammensetzung, mindestens eines 3-substituierten p-Aminophenols der Formel (I) enthält.

11. Färbezusammensetzung gemäß einem der Ansprüche 4 bis 10,
dadurch **gekennzeichnet**, daß
sie insgesamt 0,3 bis 7 Gew.%, bezogen auf das Gesamtgewicht der Zusammensetzung, Oxidationsfarbstoff-Vorstufenverbindungen vom p- und/oder o-Typ und Kuppler enthält.

12. Färbezusammensetzung gemäß einem der Ansprüche 4 bis 11,
dadurch **gekennzeichnet**, daß
sie ausserdem mindestens einen Hilfsstoff enthält, ausgewählt aus anionischen, kationischen, nicht-ionischen und amphoteren oberflächenaktiven Mitteln oder aus deren Mischungen in Mengenanteilen von 0,5 bis 55 Gew.%, aus organischen Lösungsmitteln in Konzentrationen von 1 bis 40 Gew.%, aus Verdickungsmitteln in Mengenanteilen von 0,1 bis 5 Gew.%, aus Antioxidantien in Mengenanteilen von 0,05 bis 1,5 Gew.%, aus Direktfarbstoffen, Eindringmitteln, Sequestriermitteln, Parfüm-Produkten und aus Puffermitteln, wobei die Gewichtsprozentsätze unter Bezug auf das Gesamtgewicht der Zusammensetzung angegeben sind.

13. Färbezusammensetzung gemäß einem der Ansprüche 4 bis 12,
dadurch **gekennzeichnet**, daß
sie in Form einer Flüssigkeit, Creme, eines Gels oder Schaums vorliegt und eine pH-Wert von 4 bis 11 aufweist.

14. Verfahren zur Oxidationsfärbung keratinischer Fasern und insbesondere menschlicher Haare,
dadurch **gekennzeichnet**, daß
man auf die keratinischen Fasern mindestens ein 3-substituiertes p-Aminophenol der folgenden Formel (I): 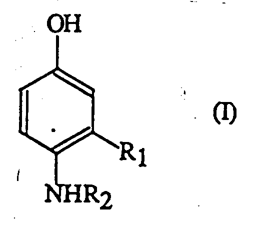 worin R₁ einen C₁₋₄-Alkyl-, C₂₋₄-Alkenyl-, C₁₋₆-Monohydroxyalkyl-, C₂₋₆-Polyhydroxyalkyl-, C₂₋₆-Alkoxyalkyl-, Nitril-, C₁₋₄-Cyanoalkyl-, C₁₋₄-Halogenalkyl- und vorzugsweise einen C₁₋₄-Fluoralkyl- sowie einen Aminoalkylrest der Formel darstellt: 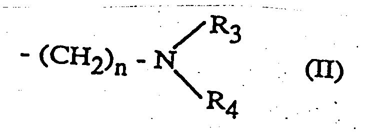 worin gilt:
n ist eine ganze Zahl von 1 bis 6;
R₃ und R₄ stellen, gleich oder verschieden, ein Wasserstoffatom oder einen C₁₋₄-Alkyl-, C₁₋₄-Hydroxyalkyl- und einen C₁₋₆-Acylrest dar;
und worin R₂ ein Wasserstoffatom, einen C₁₋₆-Alkyl-, C₁₋₆-Monohydroxyalkyl- oder einen C₂₋₆-Polyhydroxyalkylrest darstellt, mit der Maßgabe, daß:
wenn R₂ Wasserstoff ist, R₁ keinen Methyl- oder Trifluormethylrest darstellt,
oder eines seiner Säureadditionssalze, gegebenenfalls in Gegenwart eines oder mehrerer Kuppler, aufbringt und dann die Farbe mit einem oxidierenden Mittel entwickelt.

15. Verfahren gemäß Anspruch 14,
dadurch **gekennzeichnet**, daß
man, zum Zeiptunkt der Anwendung, eine Färbezusammensetzung, die, in einem zur Färbung geeigneten Medium, mindestens ein 3-substituiertes p-Aminophenol der Formel (I) und gegebenenfalls mindestens einen Kuppler enthält, mit einer oxidierenden Lösung in einer zur Entwicklung der Färbung ausreichenden Menge vermischt, wobei der pH-Wert der sich ergebenden Zusammensetzung 3 bis 11 beträgt, und daß man die so erhaltene Mischung auf die kertatinischen Fasern und insbesondere die menschlichen Haare aufbringt, sie 10 bis 40 Minuten lang verweilen und einwirken läßt, daß man dann spült, unter Schamponieren wäscht, erneut spült und trocknet.

16. Verfahren zur Färbung gemäß Anspruch 14,
dadurch **gekennzeichnet**, daß
man zu einem ersten Zeitpunkt mindestens ein 3-substituiertes p-Aminophenol der Formel (I) auf die keratinischen Fasern und zu einem zweiten Zeitpunkt eine Färbezusammensetzung aufbringt, die mindestens einen Kuppler und/oder mindestens eine weitere Oxidationsfarbstoff-Vorstufenverbindung enthält, die sich von denen der Formel (I) unterscheidet, und daß man die Färbung mit einem oxidierenden Mittel entwickelt, das in der zum zweiten Zeitpunkt aufgebrachten Zusammensetzung vorliegt oder auch auf die keratinischen Fasern zu einem dritten Zeitpunkt aufgebracht wird, daß man 10 bis 40 Minuten lang verweilen und einwirken läßt, spült, unter Schamponieren wäscht, erneut spült und dann trocknet.

17. 3-Substituiertes p-Aminophenol der Formel: 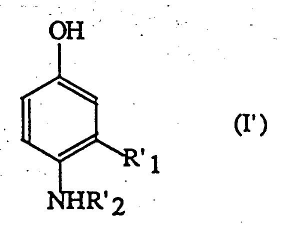 worin R₁ einen C₁₋₄-Alkyl-, C₂₋₄-Alkenyl-, C₁₋₆-Monohydroxyalkyl-, C₂₋₆-Polyhydroxyalkyl-, C₂₋₆-Alkoxyalkyl-, Nitril-, C₁₋₄-Cyanoalkyl-, C₁₋₄-Halogenalkyl- und vorzugsweise einen C₁₋₄-Fluoralkyl- sowie einen Aminoalkylrest der Formel darstellt: 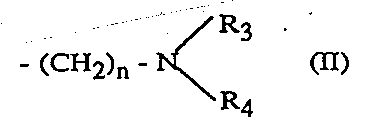 worin gilt:
n ist eine ganze Zahl von 1 bis 6;
R₃ und R₄ stellen, gleich oder verschieden, ein Wasserstoffatom oder einen C₁₋₄-Alkyl-, C₁₋₄-Hydroxyalkyl- und einen C₁₋₆-Acylrest dar;
und worin R'₂ ein Wasserstoffatom, einen C₁₋₆-Alkyl-, C₁₋₆-Monohydroxyalkyl- oder einen C₂₋₆-Polyhydroxyalkylrest darstellt,
mit der Maßgabe, daß:
- wenn R'₂ ein Wasserstoffatom bedeutet, R'₁ keinen Alkyl-, Vinyl-, Dichlormethyl- oder Trifluormethylrest darstellt;
- wenn R'₁ den Methylrest bedeutet, R'₂ keinen Ethylrest bedeutet,
oder eines seiner Additionssalze mit einer Säure.

18. 3-Substituiertes p-Aminophenol der Formel (I') gemäß Anspruch 17,
dadurch **gekennzeichnet**, daß
es aus 3-Hydroxymethyl-p-aminophenol, 3-Cyanomethyl-p-aminophenol, 3-(β-Methoxyethyl)-p-aminophenol, 3-(β-Ethoxyethyl)-p-aminophenol oder aus einem von deren Additionssalzen mit einer Säure ausgewählt ist.
